# EUROPÄISCHE PATENTSCHRIFT

(11) **EP 1 138 316 B1**
(45) Veröffentlichungstag und Bekanntmachung des Hinweises auf die Patenterteilung: **04.06.2003**
(21) Anmeldenummer: 01106800.4
(22) Anmeldetag: 19.03.2001
(51) Int. Cl.: A61K 7/13

(54) **Verfahren und Zusammensetzung zum oxidativen Färben von menschlichen Haaren**
Process and composition for oxidative dyeing of human keratinic fibres
Procédé et composition pour la coloration oxydative des fibres kératiniques humaines

(30) Priorität: 01.04.2000 DE 10016497
(43) Veröffentlichungstag der Anmeldung: 04.10.2001
(73) Patentinhaber: KPSS-Kao Professional Salon Services GmbH, 64297 Darmstadt (DE)
(72) Erfinder: Lorenz, Heribert, 64401 Gross Bieberau (DE)

(56) Entgegenhaltungen:
- EP-A- 0 642 783
- DE-A- 3 628 397
- US-A- 5 540 738
- PATENT ABSTRACTS OF JAPAN vol. 1996, no. 05, 31. Mai 1996 (1996-05-31) & JP 08 026943 A (HOYU CO LTD), 30. Januar 1996 (1996-01-30)
- PATENT ABSTRACTS OF JAPAN vol. 1997, no. 03, 31. März 1997 (1997-03-31) & JP 08 301740 A (KASHIWA KAGAKU KOGYO:KK;HEREN KAACHISU JAPAN KK), 19. November 1996 (1996-11-19)

## Beschreibung

Die vorliegende Erfindung betrifft ein Verfahren zum intensiven Färben von menschlichen Haaren unter Verwendung von Oxidationshaarfärbemitteln und Mittel zur Durchführung dieses Verfahrens.

Aus der EP 642 783 A1 ist bereits ein Haarfärbemittel auf Basis von Oxidationsfarbstoff-Vorprodukten bekannt, das die Haare innerhalb einer verkürzten Einwirkungszeit unter gleichzeitiger Aufhellung färbt und, mindestens ein näher definierten Metallsalz und mindestens eine Ammoniumverbindung, ausgewählt aus der Gruppe Ammoniumchlorid, Ammoniumsulfat, Ammoniumcarbonat, Ammoniumbicarbonat und Ammoniumcarbamat, enthält und, nach dem Vermischen mit einem Oxidationsmittel, in der gebrauchsfertigen Mischung einen pH-Wert zwischen 8 und 11, vorzugsweise 9 und 10, aufweist.

Diese Erfindung stellt eine Weiterentwicklung der aus der DE 36 28 397 C2 und der DE 36 28 398 C2 bekannten Verfahren und Mittel zum oxidativen Haarfärben dar, wo die Färbung bei einem pH-Wert zwischen 5,9 und 6,9 erfolgt und durch den Zusatz von geringen Mengen von Metallverbindungen, insbesondere Mangandioxid, Kaliumjodid, Calciumchlorid und Magnesiumsalzen zu einem schwach sauren Milieu eine befriedigende Färbung überhaupt erst ermöglicht wird.

Es wurde auch bereits vorgeschlagen, die Färbung unter Zusatz dieser Metallverbindungen auch im alkalischen Bereich durchzuführen um dadurch eine höhere Farbintensität und einer gleichzeitig verkürzten Einwirkungsdauer der auf das Haar aufgetragenen Farbmischung zu erzielen. Es hat sich jedoch gezeigt, daß im alkalischen Medium die Reaktionsgeschwindigkeit so groß ist, daß beim Vermischen von Oxidationsfarbstoffvorprodukt und Oxidationsmittel, in der Regel Wasserstoffperoxid, ein heftiges Aufschäumen entsteht und die Mischung nicht mehr ordnungsgemäß appliziert werden kann.

Es wurde nunmehr gefunden, daß dieser Effekt dadurch verhindert werden kann, daß eine mindestens eine Entwickler- und mindestens eine Kupplersubstahz und mindestens eine Metallverbindung, ausgewählt aus Mangandioxid, Kaliumjodid, Natriumjodid, Lithiumchlorid, Calciumchlorid, Calciumnitrat, Magnesiumchlorid, Magnesiumacetat, Bariumnitrat, Bariumchlorid, Kupfer(II)chlorid, Kupfer(ll)sulfat, Eisenoxid, Eisenchlorid, Cobaltchlorid, Cersulfat, Vanadiumsulfat, Kaliumbichromat und/oder Natriumbichromat, enthaltende wäßrige Oxidationsfarbstoffvorprodukt-Zusammensetzung (A), die einen alkalischen pH-Wert aufweist, mit einer wäßrigen Wasserstoffperoxidzusammensetzung (B) vermischt wird, die einen sauren pH-Wert aufweist und 0,05 bis 0,5 Gew.-%,1 -Hydroxyethan-1,1-diphosphonsäure und/oder deren Alkali- oder Ammoniumsalze enthält, wobei die gebrauchsfertige Färbemischung einen pH-Wert zwischen 7 und 12 besitzt. Dadurch ist es möglich, eine intensive, stabile Haarfärbung ohne störende Nebeneffekte zu erreichen.

Gegenstand der Erfindung ist auch ein Mittel zum oxidativen Färben von menschlichen Haaren, bestehend aus zwei bis zur Anwendung voneinander getrennt gehaltenen wäßrigen Zusammensetzungen A und B, wobei die eine Zusammensetzung A mindestens eine Entwickler- und mindestens eine Kupplersubstanz sowie mindestens eine oder mehrere Metallverbindungen, ausgewählt aus Mangandioxid, Kaliumjodid, Natriumjodid, Lithiumchlorid, Calciumchlorid, Calciumnitrat, Magnesiumchlorid, Magnesiumacetat, Bariumnitrat, Bariumchlorid, Kupfer(ll)chlorid, Kupfer(II)sulfat, Eisenoxid, Eisenchlorid, Cobaltchlorid, Cersulfat, Vanadiumsulfat, Kaliumbichromat und/oder Natriumbichromat, enthält und einen alkalischen pH-Wert aufweist, und die Zusammensetzung B Wasserstoffperoxid und 0,1 bis 5 Gew.-% 1-Hydroxyethan-1,1-diphosphonsäure und/oder deren Alkali- oder Ammoniumsalze enthält und einen sauren pH-Wert aufweist, wobei beim Vermischen dieser Zusammensetzungen A und B eine Färbemittelmischung mit einem pH-Wert zwischen 7,0 und 12,0 erhalten wird.

Der Anteil der 1-Hydroxyethan-1,1-diphosphonsäure (HEDP) in der sauren Wasserstoffperoxid-Zusammensetzung liegt vorzugsweise bei etwa 0,1 bis 2,5, insbesondere etwa 0,2 bis 1 Gew.-%. Der pH-Wert derselben liegt insbesondere zwischen etwa 1,5 bis 5, vorzugsweise 2 bis 4. Geeignete Salze der HEDP sind insbesondere die Natrium- und Ammoniumsalze.
Der anteil der Metallverbindungen in der Oxidationsfarbstoffvorprodukt-Zusammensetzung liegt insbesondere bei etwa 0,0005 bis etwa 1, vorzugsweise etwa 0,001 bis 0,5, besonders bevorzugt etwa 0,005 bis 0,25 Gew.%.

Besonders bevorzugte Metallverbindungen sind Mangandioxid, Kaliumjodid, Natriumjodid, Kupfer(II)chlorid, Kupfer(ll)sulfat, Calciumchlorid und/oder Calciumnitrat.

Die zum Einsatz gelangenden alkalisch eingestellten Oxidationsfarbstoffmischungen, die mindestens je eine Entwickler- und je eine Kupplersubstanz sowie ein Oxidationsmittel enthalten, sind an sich bekannt.

Zu diesen gebrauchsfertigen (d.h., das Peroxid enthaltenden) Mischungen, deren pH-Wert zwischen etwa 7 und 12, vorzugsweise 9 und 10, insbesondere bei etwa 9,5 liegt, wird hierzu auf den Stand der Technik, z.B. auf die Monographie von K. Schrader, "Grundlagen und Rezepturen der Kosmetika", 2. Aufl., S. 784-804 (1989), verwiesen; die dort beschriebenen Produkte sind im Rahmen des erfindungsgegemäßen Verfahrens ebenso einsetzbar wie die aus dem umfangreichen Stand der Technik bekannten weiteren Entwickler- und Kupplersubstanzen sowie Nuanceure.

Beispielhafte Entwicklersubstanzen sind insbesondere 1,4-Diaminobenzol, 2,5-Diaminotoluol, Tetraaminopyrimidine, Triaminohydroxypyrimidine, 1,2,4-Triaminobenzol, 2-(2,5-Diaminophenyl)ethanol, 2-(2-Hydroxyethylamino)-5-aminotoluol, 1-Amino-4-bis-(2'-hydroxyethyl)-aminobenzol und 4-Amino-3-methylphenol bzw. deren wasserlösliche Salze; beispielhafte Kupplersubstanzen sind Resorcin, 2-Methylresorcin, 4-Chlorresorcin, 2-Amino-4-chlorphenol, 4-(N-methyl)aminophenol, 2-Aminophenol, 3-Aminophenol, 1-M-2-hydroxy-4-aminobenzol, 3-N,N-Dimethylaminophenol, 5-Amino-2-methylphenol, 3 -Amino-2-methylamino-6-methoxypyridin, 2-Amino-3-hydroxypyridin, 4-Aminodiphenylamin, 4,4'-Diaminodophenytamin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, α-Naphthol, 1,4-Diamino-2-chlor-benzol, 4,6-Dichlorresorcin, 1,3-Diaminotoluol, 4-Hydroxy-1,2-methylendioxybenzol, 1,5-Dihydroxynaphthalin, 1,7-Dihydroxynaphthalin, 2,7-Dihydroxynaphthalin, 1-Hydroxynaphthalin, 4-Hydroxy-1,2-methylendioxybenzol, 2,4-Diamino-3-chlorphenol und/oder 1 -Methoxy-2-amino-4-(2'-hydroxyethylamino)benzol, ohne daß diese beispielhafte Aufzählung Anspruch auf Vollständigkeit erhaben könnte.

Entwickler- und Kupplersubstanzen sind vorzugsweise im Molverhältnis 1:3 bis 5:1, insbesondere etwa 1:1 und etwa 3:1, enthalten; ihr Anteil in den erfindungsgemäß eingesetzten Farbmischungen kann jeweils etwa 0,25 bis etwa 5 Gew.-%, je nach gewünschter Färbung, betragen.

Als Oxidationsmittel werden vor allem 2 bis 12%-ige Wasserstoffperoxid-Lösungen, -Emulsionen oder -Gele eingesetzt.

Die Oxidationsfarbstoffzusammensetzungen können als Lösungen, Cremes, Pasten, Gele, Aerosole, etc. Verwendung finden.

Die Anwendung erfolgt durch Vermischen der beiden Zusammensetzungen A und B, wobei das Gewichtsverhältnis, je nach Wasserstoffperoxid-Konzentration, zwischen etwa 2:1 und 1:4 liegt.

Die folgenden Beispiele illustrieren die Erfindung.

### Beispiel 1

20g einer Oxidationsfarbstoffvorprodukt-Zusammensetzung -Zusammensetzung aus

| | |
|---|---|
| Cetylstearylalkohol | 10,00 (Gew.-%) |
| Kokosfettsäuremonoethanolamid | 2,00 |
| Stearinsäuremonoethanolamid | 2,00 |
| Stearinsäurediethanolamid | 1,00 |
| p-Toluylendiaminsulfat | 0,25 |
| m-Aminophenol | 0,01 |
| Resorcin | 0,01 |
| p-Aminophenol | 0,08 |
| p-Amino-o-kresol | 0,06 |
| Pikraminsäure | 0,05 |
| Monoethanolamin | 3,20 |
| Ammoniumchlorid | 0,20 |
| Natriumlaurylsulfat | 0,30 |
| Mangan(IV)oxid | 0,06 |
| Natriumsulfit | 0,25 |
| Ethylendiaminotetraessigsäure | 0,20 |
| Parfum | 0,20 |
| Wasser | ad 100,00 |

die einen pH-Wert von 9,8 aufwies, wurden mit 40 g einer 2%-igen Wasserstoffperoxid-Lösung der folgenden Zusammensetzung (B)

| | |
|---|---|
| Wasserstoffperoxid | 2,00 (Gew.-%) |
| Polyoxyethylen-Polyoxypropylen-Blockcopolymerisat | 1,00 |
| (Poloxamer^{R}) | |
| Triethanolaminlaurylethersulfat | 0,70 |
| PEG-7-glycerylcocoat | 0,50 |
| 1-Hydroxyethan-1,1-diphosphonsäure | 0,25 |
| Wasser | ad 100,00 |

die einen pH-Wert von 2,9 aufwies, vermischt und die Mischung, deren pH-Wert bei 9,6 lag, auf aschblondes menschliches Haar aufgetragen. Nach zwanzigminütiger Einwirkung wurde gespült, gewaschen und getrocknet. Es wurde eine ausdrucksvolle haselnußblonde Färbung erhalten.

Das Weglassen von HEDP führte beim Vermischen der Zusammensetzung A und B zu einer heftigen Reaktion, die aufgrund der starken Schaumbildung kein gleichmäßiges Auftragen auf das Haar gestattete und demzufolge auch zu keiner befriedigenden Färbung führte.

### Beispiel 2

Eine Oxidationsfarbstoffvorprodukt-Zusammensetzung A, bestehend aus

| | |
|---|---|
| Cetylstearylalkohol | 10,00 (Gew.-%) |
| Kokosfettsäuremonoethanolamid | 2,00 |
| Stearinsäuremonoethanolamid | 2,00 |
| Stearinsäurediethanolamid | 1,00 |
| p-Toluylendiaminsulfat | 0,50 |
| p-Amino-o-kresol | 0,40 |
| p-Aminophenol | 0,10 |
| Monoethanolamin | 6,20 |
| Ammoniumchlorid | 0,20 |
| Natriumlaurylsulfat | 0,30 |
| Kupfer-II-chlorid | 0,0004 |
| Eisen-III-oxid | 0,000 |
| Natriumsulfit | 0,25 |
| Ethylendiaminotetraessigsäure | 0,20 |
| Parfum | 0,20 |
| Wasser | ad 100,00 |

die einen pH-Wert von 10,3 aufwies, wurden im Gewichtsverhältnis 1:1 mit einer Wasserstoffperoxid-Zusammensetzung B, bestehend aus

| | |
|---|---|
| Wasserstoffperoxid | 6,0 (Gew.-%) |
| Cetylstearylalkohol | 1,80 |
| Natriumlaurylsulfat | 0,20 |
| 1-Hydroxyethan-1,1-diphosphonsäure | 0,30 |
| Salicylsäure | 0,10 |
| Wasser | ad 100,00 |

die einen pH-Wert von 2,0 aufwies, gemischt und auf mittelblondes Haar aufgebracht (pH-Wert: 10,0).
Nach zwanzigminütiger Einwirkung, Waschen und Trocknen wurde ein Haar mit kräftigem, glänzenden Rotton erhalten.
Weglassen der 1-Hydroxyethan-1,1-diphosphonsäure führt beim Vermischen von A und B zu einer heftigen, überschäumenden Reaktion.

### Beispiel 3

Eine Oxidationsfarbstoffvorprodukt-Zusammensetzung, bestehend aus

| | |
|---|---|
| Cetylstearylalkohol | 10,00 (Gew.-%) |
| Kokosfettsäuremonoethanolamid | 2,00 |
| Stearinsäuremonoethanolamid | 2,00 |
| Stearinsäurediethanolamid | 1,00 |
| p-Toluylendiaminsulfat | 0,20 |
| p-Aminophenol | 0,70 |
| p-Amino-o-kresol | 0,70 |
| Monoethanolamin | 4,00 |
| Ammoniumchlorid | 0,20 |
| Natriumlaurylsulfat | 0,30 |
| Kaliumjodid | 0,04 |
| Mangan-IV-oxid | 0,05 |
| Natriumsulfit | 0,12 |
| Ethylendiaminotetraessigsäure | 0,20 |
| Parfum | 0,20 |
| Wasser | ad 100,00 |

mit einem pH-Wert von 10,0 wurde im Gewichtsverhältnis 1:2 mit einer Wasserstoffperoxid-Zusammensetzung, bestehend aus

| | |
|---|---|
| Wasserstoffperoxid | 4,0 (Gew.-%) |
| Polyoxyethylen-Polyoxypropylen Blockcopolymerisat | 1,0 |
| (Poloxamer^{R}) | |
| Triethanolaminlaurylethersulfat | 0,7 |
| PEG-7-glycerylcocoat | 0,5 |
| 1-Hydroxyethan-1,1-diposphonsäure | 0,3 |
| Wasser | ad 100,0 |

mit einem pH-Wert von 2,9 vermischt und die erhaltene Mischung (pH-Wert: 9,7) auf aschblondes Haar aufgebracht. Nach zwanzigminütiger Einwirkung, Waschen und
Trocknen wurde ein intensiver kupferroter Farbton erhalten.
Weglassen der HEDP ergab beim Vermischen von A und B eine heftige Reaktion, die eine gleichmäßige Ausfärbung verhinderte.

## Patentansprüche

1. Verfahren zum oxidativen Färben von menschlichen Haaren, wobei auf das Haar eine wäßrige Zusammensetzung aufgebracht wird, die einen pH- Wert zwischen 7,0 und 12,0 aufweist, **dadurch gekennzeichnet, daß** diese Zusammensetzung (AB) erhalten wurde durch Vermischen einer Oxidationsfarbstoffvorprodukt-Zusammensetzung (A), die mindestens eine Entwickler- und mindestens eine Kupplersubstanz sowie eine oder mehrere Metallverbindungen, ausgewählt aus Mangandioxid, Kaliumjodid, Natriumjodid, Lithiumchlorid, Calciumchlorid, Calciumnitrat, Magnesiumchlorid, Magnesiumacetat, Bariumnitrat, Bariumchlorid, Kupfer(ll)chlorid, Kupfer(ll)sulfat. Eisenoxid, Eisenchlorid, Cobaltchlorid, Cersulfat, Vanadiumsulfat, Kaliumbichromat und/oder Natriumbichromat, enthält und einen pH-Wert im alkalischen Bereich aufweist, und einer Wasserstoffperoxid-Zusammensetzung (B), die einen pH-Wert im sauren Bereich aufweist, und 0,05 bis 5,0 Gew.-%, berechnet auf die Zusammensetzung (B), 1-Hydroxyethan-1,1-diphosphonsäure und/oder deren Alkali- oder Ammoniumsalze enthält.

2. Verfahren nach Anspruch 1, **dadurch gekennzeichnet, daß** die Wasserstoffperoxid-Zusammensetzung (B) einen pH-Wert von 1,5 bis 5 aufweist.

3. Verfahren nach Anspruch 2, **dadurch gekennzeichnet, daß** die Wasserstoffperoxid-Zusammensetzung (B) einen pH-Wert von 2 bis 4 aufweist.

4. Mittel zum oxidativen Färben von menschlichen Haaren, bestehend aus zwei bis zur Anwendung voneinander getrennt gehaltenen wäßrigen Zusammensetzungen A und B, wobei die eine Zusammensetzung A mindestens eine Entwickler- und mindestens eine Kupplersubstanz sowie mindestens eine oder mehrere Metallverbindungen, ausgewählt aus Mangandioxid, Kaliumjodid, Natriumjodid, Lithiumchlorid, Calciumchlorid, Calciumnitrat, Magnesiumchlorid, Magnesiumacetat, Bariumnitrat, Bariumchlorid, Kupfer(II)chlorid, Kupfer(ll)sulfat, Eisenoxid, Eisenchlorid, Cobaltchlorid, Cersulfat, Vanadiumsulfat, Kaliumbichromat und/oder Natriumbichromat, enthält und einen alkalischen pH-Wert aufweist, und die Zusammensetzung B Wasserstoffperoxid und 0,1 bis 5 Gew.-% 1-Hydroxyethan-1,1-diphosphonsäure und/oder deren Alkali- oder Ammoniumsalze enthält und einen sauren pH-Wert aufweist, wobei beim Vermischen dieser Zusammensetzungen A und B eine Färbemittelmischung mit einem pH-Wert zwischen 7,0 und 12,0 erhalten wird.

5. Verfahren und Mittel nach einem oder mehreren der Ansprüche 1 bis 4, enthaltend als Metallverbindung Mangandioxid.

6. Verfahren und Mittel nach einer oder mehreren der Ansprüche 1 bis 4, enthaltend als Metallverbindung Kalium- undloder Natriumjodid.

7. Verfahren und Mittel nach einer oder mehreren der Ansprüche 1 bis 4, enthaltend als Metallverbindung Kupfer(ll)chlorid und/oder Kupfer(II)sulfat.

8. Verfahren und Mittel nach einer oder mehreren der Ansprüche 1 bis 4, enthaltend als Metallverbindung Calciumchlorid und/oder Calciumnitrat.

## Claims

1. Process for the oxidative dyeing of human hair, whereby an aqueous composition having a pH-value between 7.0 and 12.0 is applied to the hair, wherein this Composition (AB) was obtained by mixing of an oxidation dyestuff precursor Composition (A), which comprises at least one developing and at least one coupling substance, as well as one or more metal compounds selected from manganese dioxide, potassium iodide, sodium iodide, lithium chloride, calcium chloride, calcium nitrate, magnesium chloride, barium nitrate, barium chloride, copper (ll) chloride, copper (lll) sulphate, iron oxide, iron chloride, cobalt chloride, cerium sulphate, vanadium sulphate, potassium bichromate and/or sodium bichromate and having an alkaline pH-value, and a hydrogen peroxide Composition (B), having a pH-value in the acidic range and comprising 0.05 & to 5.0 % by weight, calculated to the total composition, of 1-hydroxyethane-1 ,1-diphosphonic acid and/or the alkali or ammonium salts thereof.

2. Process according to claim 1, wherein the hydrogen peroxide Composition (B) has a pH-value of 1.5 to 5.

3. Process according to claim 2, wherein the hydrogen peroxide Composition (B) has a pH-value of 2 to 4.

4. Composition for the oxidative dyeing of human hair, consisting of two aqueous Compositions A and B stored separately until application, whereby the Composition A comprises at least one developing and one coupling substance as well as at least one or more metal compounds selected from manganese dioxide, potassium iodide, sodium iodide, lithium chloride, calcium chloride, calcium nitrate, magnesium chloride, barium nitrate, barium chloride, copper (ll) chloride, copper (lll) sulphate, iron oxide, iron chloride, cobalt chloride, cerium sulphate, vanadium sulphate, potassium bichromate and/or sodium bichromate, having an alkaline pH-value, and the Composition B comprises hydrogen peroxide and 0.1 % to 5 % by weight, calculated to the total composition, of 1-hydroxyethane-1,1-diphosphonic acid and/or the alkali or ammonium salts thereof and having an acidic pH-value, whereby mixing of these Compositions A and B results in a hair-dyeing composition with a pH-value between 7.0 and 12.0.

5. Process and composition according to one or more of claims 1 to 5, comprising manganese dioxide as metal compound.

6. Process and composition according to one or more of claims 1 to 5, containing potassium or sodium iodide as metal compound.

7. Process and composition according to one or more of claims 1 to 5, comprising copper(ll) chloride and/or copper(ll) sulphate as metal compound.

8. Process and composition according to one or more of claims 1 to 5, containing calcium chloride and/or calcium nitrate as metal compound.

## Revendications

1. Procédé pour la coloration par oxydation de cheveux humains par application d'une composition aqueuse qui présente un pH entre 7,0 et 12,0, **caractérisé en ce que** cette composition (AB) a été obtenue par mélange d'une composition de précurseurs de colorants d'oxydation (A) qui contient au moins une substance de développement et au moins une substance de couplage ainsi qu'un ou plusieurs composés métalliques sélectionnés parmi le dioxyde de manganèse, l'iodure de potassium, l'iodure de sodium, le chlorure de lithium, le chlorure de calcium, le nitrate de calcium, le chlorure de magnésium, l'acétate de magnésium, le nitrate de baryum, le chlorure de baryum, le chlorure de cuivre II, le sulfate de cuivre II, l'oxyde de fer, le chlorure de fer, le chlorure de cobalt, le sulfate de cérium, le sulfate de vanadium, le bichromate de potassium et/ou le bichromate de sodium, et qui présente un pH basique, avec une composition de peroxyde d'hydrogène (B) qui présente un pH acide et qui contient un pourcentage en poids calculé par rapport à la composition (B) de 0,05 à 5,0 d'acide 1-hydroxyéthane-1,1-diphosphonique et/ou de ses sels d'ammonium ou alcalins.

2. Procédé selon la revendication 1, **caractérisé en ce que** la composition de peroxyde d'hydrogène (B) présente un pH de 1,5 à 5.

3. Procédé selon la revendication 2, **caractérisé en ce que** la composition de peroxyde d'hydrogène (B) présente un pH de 2 à 4.

4. Produit de coloration par oxydation de cheveux humains, composé de deux compositions aqueuses A et B maintenues séparément l'une de l'autre jusqu'à l'utilisation, la composition A contenant au moins une substance de développement et au moins une substance de couplage ainsi qu'au moins un ou plusieurs composés métalliques, sélectionnés parmi le dioxyde de manganèse, l'iodure de potassium, l'iodure de sodium, le chlorure de lithium, le chlorure de calcium, le nitrate de calcium, le chlorure de magnésium, l'acétate de magnésium, le nitrate de baryum, le chlorure de baryum, le chlorure de cuivre II, le sulfate de cuivre II, l'oxyde de fer, le chlorure de fer, le chlorure de cobalt, le sulfate de cérium, le sulfate de vanadium, le bichromate de potassium et/ou le bichromate de sodium, et qui présente un pH alcalin, et la composition B contient du peroxyde d'hydrogène et un pourcentage en poids de 0,1 à 5 % d'acide 1-hydroxyethane-1,1-diphosphonique et/ou de ses sels d'ammonium ou alcalins et présente un pH acide, moyennant quoi en mélangeant ces compositions A et B on obtient un mélange de colorants ayant un pH entre 7,0 et 12,0.

5. Procédé et produit selon l'une ou plusieurs des revendications 1 à 4, comprenant du dioxyde de manganèse comme composé métallique.

6. Procédé et produit selon une ou plusieurs des revendications 1 à 4, comprenant du iodure de potassium et/ou de sodium comme composé métallique.

7. Procédé et produit selon une ou plusieurs des revendications 1 à 4, comprenant du chlorure de cuivre II et/ou du sulfate de cuivre II comme composé métallique.

8. Procédé et produit selon un ou plusieurs revendications 1 à 4, comprenant du chlorure de calcium et/ou du nitrate de calcium comme composé métallique.
